(19) Europäisches Patentamt
European Patent Office
Office européen des brevets

(11) EP 4 625 436 A1

(12) EUROPEAN PATENT APPLICATION
published in accordance with Art. 153(4) EPC

(43) Date of publication:
01.10.2025 Bulletin 2025/40

(51) International Patent Classification (IPC):
G16H 50/30 (2018.01)        A61B 5/366 (2021.01)
A61B 5/346 (2021.01)        A61B 5/00 (2006.01)

(21) Application number: 23893018.4

(22) Date of filing: 11.05.2023

(86) International application number:
PCT/CN2023/093494

(87) International publication number:
WO 2024/108917 (30.05.2024 Gazette 2024/22)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 21.11.2022 CN 202211451652

(71) Applicant: Hyperbio Biological Technology Co.,
Ltd
Changsha, Hunan 410000 (CN)

(72) Inventors:
• LI, Xiaoqin
  Changsha, Hunan 410000 (CN)
• HUANG, Qingxi
  Changsha, Hunan 410000 (CN)
• LONG, Wenyao
  Changsha, Hunan 410000 (CN)

(74) Representative: Studio Torta S.p.A.
Via Viotti, 9
10121 Torino (IT)

(54) **ELECTROCARDIOGRAM SIGNAL PROCESSING METHOD AND APPARATUS, AND RELATED DEVICE**

(57) The present application relates to an electrocardiogram signal processing method and apparatus, and a related device. The method comprises: acquiring electrocardiosignals of a first preset number of leads in a resting state, the leads comprising a second preset number of limb leads, and the second preset number being smaller than the first preset number; performing data processing on each electrocardiosignal in the resting state so as to obtain a static high-frequency QRS waveform feature set, the static high-frequency QRS waveform feature set comprising a QRS time limit, the number of first positive indexes, the number of first target leads and the number of target limb leads; and performing assessment and analysis on a risk assessment feature set so as to obtain a heart failure risk assessment score, the heart failure risk assessment score being used for judging the risk level of developing heart failure, and the risk assessment feature set comprising said static high-frequency QRS waveform feature set.

FIG. 2

**Description**

CROSS-REFERENCE TO RELATED APPLICATION

This application claims the priority to Chinese Patent Application No. 2022114516528, titled "ELECTROCARDIOGRAM SIGNAL PROCESSING METHOD AND APPARATUS, AND RELATED DEVICE", filed on November 21, 2022 with the China National Intellectual Property Administration, which is incorporated herein by reference in its entirety.

**FIELD**

The present disclosure relates to the technical field of electrocardiogram signal processing, and in particular to a method and an apparatus for processing electrocardiogram signals, a computer device and a storage medium.

**BACKGROUND**

With the development of social economy, people's living standards are also increasing year by year. More and more people are transitioning from manual labor to mental labor, which leads to a decrease in the average exercise time of people year by year. Many diseases that used to be prevalent among the elderly are gradually appearing more and more frequently for young people, such as myocardial infarction and heart failure.

In conventional methods, a risk of heart failure is usually assessed jointly by testing blood and measuring detection data such as N-Terminal Pro-Brain Natriuretic Peptide (NT-proBNP), plasma homocysteine, Left Ventricular Ejection Fraction (LVEF) of echocardiography, Left Ventricular End Systolic Diameter (LVESD), and 6-minute walk test result. However, the risk assessment of heart failure with the above conventional methods is time-consuming and results in inaccurate assessment result.

**SUMMARY**

According to the embodiments of the present disclosure, a method and an apparatus for processing electrocardiogram signals and a related device are provided.

In a first aspect, a method for processing electrocardiogram signals is provided and the method includes:

acquiring electrocardiogram signals in a resting state from a first preset quantity of leads; where the leads include a second preset quantity of limb leads; and the second preset quantity is less than the first preset quantity;

performing data processing on the electrocardiogram signals in the resting state to obtain a static high-frequency QRS waveform feature set; where the static high-frequency QRS waveform feature set includes a QRS duration, a quantity of a first positive index, a quantity of a first target lead and a quantity of a target limb lead; for each of the leads, the first positive index is for characterizing that a high-frequency morphological index corresponding to the lead is greater than a first threshold; the high-frequency morphological index is a ratio of a first total area to a second total area corresponding to the lead; the first total area is a total area of a reduced amplitude zone of the electrocardiogram signal in the resting state corresponding to the lead; the second total area is a total area under an envelope of the electrocardiogram signal in the resting state corresponding to the lead; the first target lead is a lead for which a total quantity of times the electrocardiogram signal in the resting state is in a downward trend exceeds a second threshold, and the target limb lead is a limb lead for which a QRS complex voltage of the electrocardiogram signals in the resting state is less than a third threshold;

assessing and analyzing a risk assessment feature set to obtain a risk assessment score of heart failure, where the risk assessment score of heart failure is for determining a risk magnitude of heart failure and the risk assessment feature set includes the static high-frequency QRS waveform feature set.

In an embodiment, the risk assessment feature set further includes a dynamic high-frequency QRS waveform feature set and the method further includes: acquiring electrocardiogram signals in a stress state from the leads; performing data processing on the electrocardiogram signals in the stress state to obtain respective high-frequency waveform curves; performing feature analysis on the respective high-frequency waveform curves to obtain the dynamic high-frequency QRS waveform feature set, where the dynamic high-frequency QRS waveform feature set includes at least two of a quantity of a second positive index, an initial average voltage, a maximum output power, and a quantity of a second target lead; the second positive index is for characterizing that a relative amplitude reduction value corresponding to the lead is greater than a fourth threshold; the relative amplitude reduction value is a ratio of an absolute amplitude value to a maximum RMS voltage; the absolute amplitude value is a difference between the maximum RMS voltage and a target RMS voltage; the maximum RMS voltage is a maximum of RMS voltages of each of the respective high-frequency waveform curves; the target RMS voltage is a minimum RMS voltage corresponding to a time instant later than a time instant corresponding to the maximum RMS voltage; the initial average voltage is an average of initial voltages of the respective high-frequency waveform curves; the maximum output power is determined based on the maximum of the root-mean-square voltages of the high-frequency waveform curves; the second target lead is a lead whose the high-frequency waveform curve presents a target waveform; and the target waveforms includes a U wave, an L wave and a small V wave.

In an embodiment, the performing data processing on the electrocardiogram signals in the resting state to obtain the static high-frequency QRS waveform feature set includes: for each of the leads, performing data extraction on the electrocardiogram signal in the resting state to obtain a high-frequency component of a static QRS waveform corresponding to the lead; determining a reduced amplitude zone corresponding to the lead based on the high-frequency component of the static QRS waveform, and calculating a first total area and a second total area of the lead; acquiring a high-frequency morphological index of the lead based on a ratio of the first total area to the second total area; and counting the quantity of the first positive index based on the high-frequency morphological indexes of all the leads and the first threshold.

In an embodiment, after the determining the reduced amplitude zone corresponding to the lead based on the high-frequency component of the static QRS waveform, the method further includes: calculating an area of the reduced amplitude zone corresponding to the lead; counting a quantity of a target area of the lead based on the area of the reduced amplitude zone corresponding to the lead; where the target area refers to an area of the reduced amplitude zone that is greater than a fifth threshold; determining the quantity of the target area of the lead as the total quantity of times corresponding to the lead; and determining the quantity of the first target lead based on the total quantities of times corresponding to all the leads and the second threshold.

In an embodiment, the assessing and analyzing the risk assessment feature set to obtain the risk assessment score of heart failure includes: inputting the risk assessment feature set into a preset risk assessment function or a pre-trained risk assessment network model to obtain the risk assessment score of heart failure.

In an embodiment, the above method further includes: determining a risk level of heart failure based on the risk assessment score of heart failure; and outputting monitoring and early-warning data of heart failure in response to the risk level of heart failure being greater than a level threshold.

In a second aspect, an apparatus for processing electrocardiogram signals is provided and the apparatus includes a signal acquisition module, a signal processing module and an assessment and analysis module.

The signal acquisition module is configured to acquire electrocardiogram signals in a resting state from a first preset quantity of leads, where the leads include a second preset quantity of limb leads, and the second preset quantity is less than the first preset quantity.

The signal processing module is configured to perform data processing on the electrocardiogram signals in the resting state to obtain a static high-frequency QRS waveform feature set, where the static high-frequency QRS waveform feature set includes a QRS duration, a quantity of a first positive index, a quantity of a first target lead and a quantity of a target limb lead; the first positive index is for characterizing that a high-frequency morphological index corresponding to the leads is greater than a first threshold; the high-frequency morphological index is a ratio of a first total area to a second total area corresponding to each of the leads; the first total area is a total area of reduced amplitude zones of the electrocardiogram signals in the resting state corresponding to each of the leads; the second total area is a total area under an envelope of the electrocardiogram signals in the resting state corresponding to each of the leads; the first target lead is a lead for which a total quantity of times the electrocardiogram signal in the resting state is in a downward trend exceeds a second threshold, and the target limb lead is a limb lead for which a QRS complex voltage of the electrocardiogram signals in the resting state is less than a third threshold.

The assessment and analysis module is configured to assess and analyze a risk assessment feature set to obtain a risk assessment score of heart failure, where the risk assessment score of heart failure is for determining a risk magnitude of heart failure, and the risk assessment feature set includes the static high-frequency QRS waveform feature set.

In a third aspect, a computer device is provided. The computer device includes a memory and a processor. The memory stores computer-readable instructions, and the computer-readable instructions, when being executed by the processor, perform the method according to any one of the above embodiments of the method.

In a fourth aspect, a system for processing electrocardiogram signals is provided. The system includes an electrocardiogram signal acquisition device and the computer device according to any one of the above embodiments of the device. The electrocardiogram signal acquisition device is electrically connected to the computer device and is configured to acquire electrocardiogram signals in the resting state.

In a fifth aspect, a computer-readable storage medium is provided. The computer-readable storage medium stores computer-readable instructions. The computer-readable instructions, when being executed by a processor, perform the method according to any one of the above embodiments of the method.

Details of one or more embodiments of the present disclosure are provided in the drawings and descriptions below. Other features and advantages of the present disclosure are clarified in the specification, the drawings, and the claims.

## BRIEF DESCRIPTION OF THE DRAWINGS

For more clearly illustrating technical solutions in embodiments of the present disclosure, drawings referred to describe the embodiments are briefly described hereinafter. Apparently, the drawings in the following description are only some embodiments of the present disclosure, and for those skilled in the art, other drawings may be acquired based on these drawings without any creative efforts.

FIG. 1 is a diagram showing an application environment of a method for processing electrocardiogram signals according to an embodiment of the present disclosure;

FIG. 2 is a schematic flowchart of a method for processing electrocardiogram signals according to a first embodiment of the present disclosure;

FIG. 3 is a schematic flowchart of a step of performing data processing on electrocardiogram signals in a resting state to obtain a static high-frequency QRS waveform feature set according to a first embodiment of the present disclosure;

FIG. 4 is a schematic flowchart of a step of performing data processing on electrocardiogram signals in a resting state to obtain a static high-frequency QRS waveform feature set according to a second embodiment of the present disclosure;

FIG. 5 is a schematic flowchart of a method for processing electrocardiogram signals according to a second embodiment of the present disclosure;

FIG. 6 is a schematic flowchart of a method for processing electrocardiogram signals according to a third embodiment of the present disclosure;

FIG. 7 is a schematic flowchart of a method for processing electrocardiogram signals according to a fourth embodiment of the present disclosure;

FIG. 8 is a structural block diagram of an apparatus for processing electrocardiogram signals according to an embodiment of the present disclosure;

FIG. 9 is a diagram illustrating an internal structure of a computer device according to an embodiment of the present disclosure;

FIG. 10 is a diagram illustrating an internal structure of a system for processing electrocardiogram signals according to an embodiment of the present disclosure; and

FIG. 11 is a schematic diagram of a reduced amplitude zone according to an embodiment of the present disclosure.

## DETAILED DESCRIPTION

Hereinafter the present disclosure is described in detail in conjunction with the drawings and embodiments, in order to illustrate and clarify technical solutions, and advantages of the present disclosure. It should be understood that the embodiments described herein are only for explaining the present disclosure, and are not intended to limit the present disclosure.

In order to facilitate understanding of the present disclosure, hereinafter the present disclosure is described in detail in conjunction with the drawings. The drawings show embodiments of the present disclosure. The present disclosure may be implemented in various forms and is not limited to the embodiments described herein. On the contrary, the purpose of providing these embodiments is to make the understanding of the present disclosure more thorough and comprehensive.

Unless otherwise defined, all technical and scientific terms used herein have the same meanings as commonly understood by those skilled in the art. The terms are merely intended for describing specific embodiments, rather than limiting the present disclosure.

When used herein, "a", "an" and "said/the" in singular forms may further include plural forms, unless the context clearly indicates otherwise. It should also be understood that the terms "include/comprise" or "have" specify the presence of stated features, entireties, steps, operations, components, parts or combinations thereof, but do not exclude the possibility of the presence or addition of one or more other features, entireties, steps, operations, components, parts or combinations thereof. Meanwhile, terms "and/or" used in this specification include any and all combinations of the items listed.

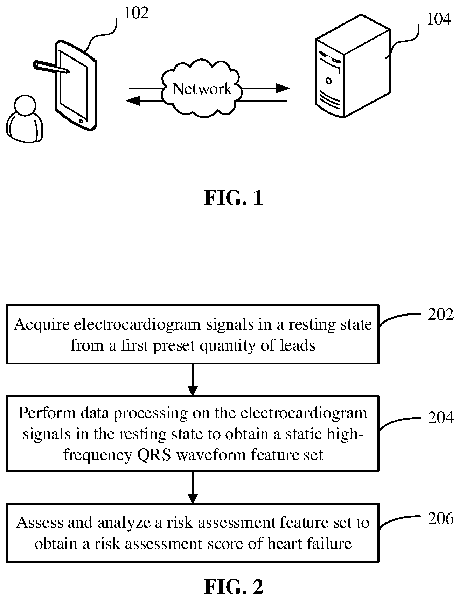

A method for processing electrocardiogram signals according to present disclosure may be applied in the application environment as shown in FIG. 1. A terminal 102 communicates with a server 104 via a network. The terminal 102 may be, but is not limited to, a personal computer, a laptop, a smartphone, a tablet computer and a portable wearable device. The server 104 may be an independent server or a server cluster including multiple servers.

In a first aspect, as shown in FIG. 2, a method for processing electrocardiogram signals is provided. Taking the application of this method to the terminal in FIG. 1 as an example for illustration, the method includes the following steps 202 to 206.

In step 202, electrocardiogram signals in a resting state are acquired from a first preset quantity of leads.

The first preset quantity of leads includes a second preset quantity of limb leads, and the second preset quantity is less than the first preset quantity. An electrocardiogram signal acquisition device electrically connected to the terminal is configured to acquire the electrocardiogram signals in the resting state from the first preset quantity of leads, and thus the terminal may acquire, through the electrocardiogram signal acquisition device, the electrocardiogram signals in the resting state from the first preset quantity of leads.

In a specific example, the first preset quantity of leads further includes a third preset quantity of chest leads. The third preset quantity is a difference between the first preset quantity and the second preset quantity. It should be understood that the first preset quantity of leads may be 12 leads, which include 6 chest leads and 6 limb leads. Alternatively, the first preset quantity of leads may be 18 leads, which include 12 chest leads and 6 limb leads. Specific examples are described above, and may be flexibly applied according to user requirements in practical applications, which are not limited herein.

In step 204, data processing is performed on the electrocardiogram signals in the resting state to obtain a static high-frequency QRS waveform feature set.

The static high-frequency QRS waveform feature set includes a QRS duration, a quantity of a first positive index, a quantity of a first target lead and a quantity of a target limb lead.

In an embodiment, the QRS duration refers to a duration from a starting point to an ending point of q wave, r wave and s wave in an electrocardiogram generated based on the electrocardiogram signals in the resting state from the leads, and is used to characterize an overall cardiac function condition corresponding to the electrocardiogram signals in the resting state from the leads.

In a specific example, a QRS duration threshold may be but is not limited to 0.12 seconds. In a case that the QRS duration is greater than the QRS duration threshold, it is characterized that the electrocardiogram generated based on the electrocardiogram signals in the resting state from the leads is abnormal. In a case that the QRS duration is less than or equal to the QRS duration threshold, it is characterized that the electrocardiogram generated based on the electrocardiogram signals in the resting state from the leads is normal. Specific examples are described above, and may be flexibly applied according to user requirements in practical applications, which are not limited herein.

In an embodiment, a high-frequency morphological index is a ratio of a first total area to a second total area corresponding to each of the leads. The first total area is a total area of a reduced amplitude zone of the electrocardiogram signal in the resting state. The second total area is a total area under an envelope of the electrocardiogram signal in the resting state. The first positive index characterizes that the high-frequency morphological index corresponding to the lead is greater than a first threshold, that is, it characterizes that the electrocardiogram signal in the resting state corresponding to the lead is abnormal. Conversely, a first negative index characterizes that a high-frequency morphological index corresponding to the lead is less than or equal to the first threshold, that is, it characterizes that the electrocardiogram signal in the resting state corresponding to the lead is normal. A quantity of the first positive index characterizes an overall degree of myocardial ischemia corresponding to the electrocardiogram signals in the resting state from the leads. That is, a large quantity of the first positive index indicates a great overall risk of myocardial ischemia corresponding to electrocardiogram signals in the resting state from the leads.

In a specific example, the first threshold may be determined based on the age of an object from which electrocardiogram signals are collected. If the age of the object is greater than 50 years old, the first threshold may be but is not limited to 8%. If the age of the object is less than or equal to 50 years old, the first threshold may be but is not limited to 15%. Alternatively, the first threshold may be the average of target high-frequency morphological indexes or 20% to further improve the accuracy of a risk assessment score of heart failure. The target high-frequency morphological indexes refer to the top three high-frequency morphological indexes in a case that high-frequency morphological indexes are sorted by values in a descending order. Specific examples are described above, and may be flexibly applied according to user requirements in practical applications, which are not limited herein.

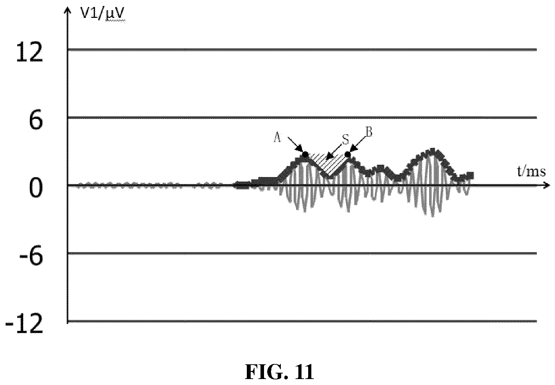

In an embodiment, the first target lead is a lead for which a total quantity of times the electrocardiogram signal in the resting state is in a downward trend exceeds a second threshold. That is, in a case that the total quantity of times the electrocardiogram signal in the resting state is in a downward trend exceeds the second threshold, a lead corresponding to the electrocardiogram signal in the resting state is determined as the first target lead. A quantity of the first target lead characterizes the overall risk of heart failure corresponding to the electrocardiogram signals in the resting state from the leads. Furthermore, the electrocardiogram signal in the resting state being in a downward trend characterizes appearance of a reduced amplitude zone (RAZ) in QRS waveform corresponding to electrocardiogram signal in the resting state. As shown in FIG. 11, the reduced amplitude zone refers to a concave zone S formed between a point A and point B in the QRS waveform corresponding to the electrocardiogram signal in the resting state. In a specific example, by counting the peak-to-peak value of the reduced amplitude zone corresponding to the electrocardiogram signal in the resting state, the total quantity of the peak-to-peak value corresponding to the electrocardiogram signal in the resting state may be determined as the total quantity of times the electrocardiogram signal in the resting state is in a downward trend. The total quantity of times the electrocardiogram signal in the resting state corresponding to each lead is in a downward trend is counted. After the total quantity of times the electrocardiogram signal in the resting state corresponding to each lead is in a downward trend is compared with the second threshold, the quantity of the lead for which the total quantity of times the electrocardiogram signal in the resting state is in a downward trend exceeds the second threshold, that is, the quantity of the first target lead, may be acquired. Specific examples are described above, and may be flexibly applied according to user requirements in practical applications, which are not limited herein.

In an embodiment, the target limb lead is a limb lead for which a QRS complex voltage of the electrocardiogram signal in the resting state is less than a third threshold. That is, in a case that the QRS complex voltage of the electrocardiogram signal in the resting state is less than the third threshold, a lead corresponding to the electrocardiogram signal in the resting state is determined as the target limb lead. A quantity of the target limb lead characterizes the overall risk of aggravated heart failure corresponding to the electrocardiogram signals in the resting state from the leads.

In a specific example, the third threshold is determined based on QRS complex voltages of the electrocardiogram signals in the resting state corresponding to the chest leads. Specific examples are described above, and may be flexibly applied according to user requirements in practical applications, which are not limited herein.

It should be understood that data processing is performed on the electrocardiogram signals in the resting state to obtain the static high-frequency QRS waveform feature set including the QRS duration, the quantity of the first positive index, the quantity of the first target lead and the quantity of the target limb lead.

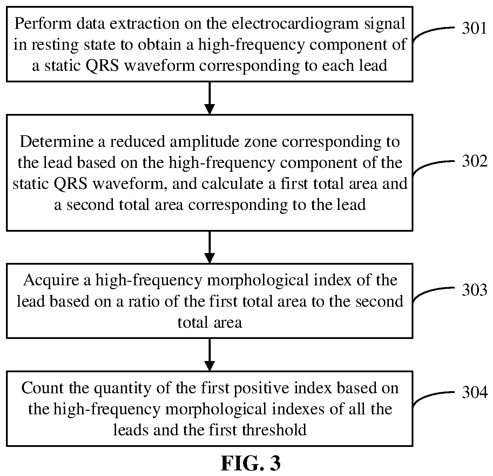

In an embodiment, as shown in FIG. 3, the step of performing data processing on the electrocardiogram signals in the resting state to obtain the static high-frequency QRS waveform feature set includes steps 301 to 304.

In step 301, data extraction is performed on the electrocardiogram signals in the resting state to obtain high-frequency components of static QRS waveforms corresponding to the leads.

In step 302, reduced amplitude zones corresponding to the leads are determined based on the high-frequency components of the static QRS waveforms corresponding to the leads, and a first total area and a second total area of each of the leads are calculated.

In step 303, a high-frequency morphological index of each of the leads is acquired based on a ratio of the first total area to the second total area of the lead.

In step 304, the quantity of the first positive index is counted based on the high-frequency morphological indexes of the leads and the first threshold.

The terminal may perform data extraction on the electrocardiogram signals in the resting state to obtain the high-frequency components of the static QRS waveforms corresponding to the leads, then determine reduced amplitude zones corresponding to leads based on the high-frequency components of the static QRS waveforms of the leads and calculate the first total area and the second total area corresponding to each of the leads based on the reduced amplitude zone corresponding to the lead, subsequently calculate the ratio of the first total area to the second total area corresponding to each lead to obtain high-frequency morphological indexes corresponding to the leads, and finally count the quantity of the first positive index based on a comparison result between the high-frequency morphological index of each lead and the first threshold.

In this embodiment, by performing data extraction on the electrocardiogram signals in the resting state to obtain the high-frequency components of the static QRS waveforms corresponding to the leads, the quantity of the first positive index can be accurately acquired based on the high-frequency components of the static QRS waveforms of the leads. Thus, a quantity of a lead for which the electrocardiogram signal in the resting state corresponding to the lead is abnormal is known based on the quantity of the first positive index, thereby acquiring the risk assessment feature set and the risk assessment score of heart failure.

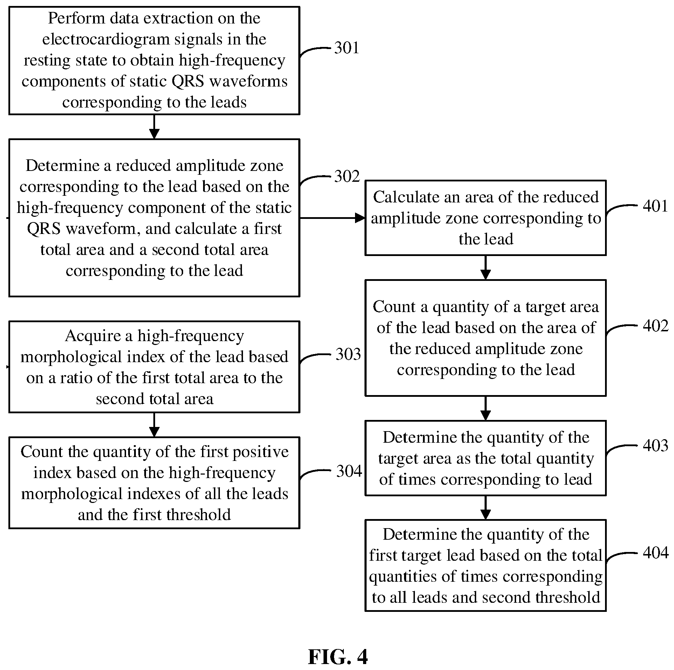

In an embodiment, as shown in FIG. 4, after the step of determining reduced amplitude zones corresponding to leads based on the high-frequency components of the static QRS waveforms of the leads, the method further includes steps 401 to 404.

In step 401, an area of the reduced amplitude zone corresponding to each of the leads is calculated.

In step 402, a quantity of a target area of the lead is counted based on the area of the reduced amplitude zone corresponding to the lead.

In step 403, the quantity of the target area of the lead is determined as the total quantity of times corresponding to the lead.

In step 404, the quantity of the first target lead is determined based on the total quantities of times corresponding to all the leads and the second threshold.

The target area refers to an area of the reduced amplitude zone that is greater than a fifth threshold. The terminal determines reduced amplitude zones corresponding to the leads based on the high-frequency components of the static QRS waveforms of the leads, and thus calculates the areas of the reduced amplitude zones corresponding to the leads. The area of the reduced amplitude zone that is greater than the fifth threshold may be determined as the target area. Then, the terminal counts the quantity of the target area of the lead based on the area of the reduced amplitude zone corresponding to the lead, and determines the quantity of the target area of the lead as the total quantity of times the electrocardiogram signal in the resting state corresponding to the lead is in a downward trend. Finally, the terminal may determine the quantity of the first target lead based on a comparison result between the total quantity of times of each of the leads and the second threshold.

In this embodiment, the quantity of the target area of the lead is counted based on the area of the reduced amplitude zone corresponding to each of the leads, so that the total quantity of times the electrocardiogram signal in the resting state is in a downward trend is accurately acquired, thus determining the quantity of the first target lead, improving the convenience of acquiring the risk assessment feature set and the risk assessment score of heart failure.

In step 206, the risk assessment feature set is assessed and analyzed to obtain the risk assessment score of heart failure.

The risk assessment score of heart failure is used to determine a risk magnitude of heart failure. The risk assessment feature set includes the static high-frequency QRS waveform feature set. The terminal assesses and analyzes the risk assessment feature set to obtain the risk assessment score of heart failure, thereby accurately assessing the risk magnitude of heart failure.

In view of this, in the above method for processing electrocardiogram signals, electrocardiogram signals in the resting state are acquired from a first preset quantity of leads; then the data processing is performed on the electrocardiogram signals in the resting state to obtain a static high-frequency QRS waveform feature set; and subsequently a risk assessment feature set is assessed and analyzed to obtain a risk assessment score of heart failure, so that the risk magnitude of heart failure can be accurately assessed. Furthermore, a complete and comprehensive static high-frequency QRS waveform feature set and a risk assessment feature set can be acquired by processing the electrocardiogram signals, and the risk magnitude of heart failure can be also directly assessed based on the risk assessment score of heart failure, instead of the risk assessment of heart failure using multiple detection data in the conventional methods, thereby reducing a time consumption of the risk assessment of heart failure and an improving the efficiency of the risk assessment of heart failure.

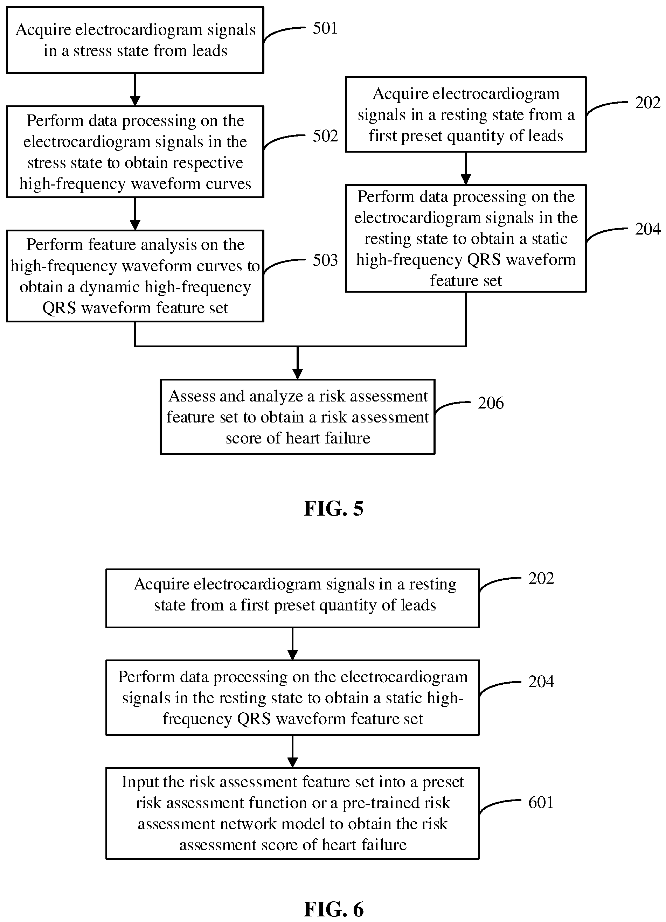

In an embodiment, as shown in FIG. 5, the risk assessment feature set further includes a dynamic high-frequency QRS waveform feature set. The method further includes steps 501 to 503.

In step 501, electrocardiogram signals in a stress state are acquired from the leads.

In step 502, data processing is performed on the electrocardiogram signals in the stress state to obtain respective high-frequency waveform curves.

In step 503, feature analysis is performed on the high-frequency waveform curves to obtain a dynamic high-frequency QRS waveform feature set.

The electrocardiogram signals in the stress state include electrocardiogram signals in the resting state, an exercise state and a recovery state. An electrocardiogram signal acquisition device electrically connected to the terminal is configured to acquire the electrocardiogram signals in the stress state from the first preset quantity of leads. The terminal may acquire, through the electrocardiogram signal acquisition device, the electrocardiogram signals in the stress state from the first preset quantity of leads, then perform data processing on the electrocardiogram signals in the stress state from the leads to obtain respective high-frequency waveform curves, and subsequently perform feature analysis on the high-frequency waveform curves to obtain the dynamic high-frequency QRS waveform feature set.

In a specific example, the step of performing feature analysis on the high-frequency waveform curves to obtain the dynamic high-frequency QRS waveform feature set includes: extracting feature points from the high-frequency waveform curves to obtain respective target feature points; extracting features of the high-frequency waveform curves based on the respective target feature points to obtain the dynamic high-frequency QRS waveform feature set. Specific examples are described above, and may be flexibly applied according to user requirements in practical applications, which are not limited herein.

In an embodiment, the dynamic high-frequency QRS waveform feature set includes at least two of a quantity of a second positive index, an initial average voltage, a maximum output power, and a quantity of a second target lead. It should be understood that if a quantity of the features in the dynamic high-frequency QRS waveform feature set is large, the data for assessing and analyzing the risk assessment feature set is more comprehensive and the risk assessment score of heart failure is more accurate.

A relative amplitude reduction value is a ratio of an absolute amplitude value to a maximum RMS voltage. The absolute amplitude value is a difference between the maximum RMS voltage and a target RMS voltage. The maximum RMS voltage is a maximum of the RMS voltages of each high-frequency waveform curve. The target RMS voltage is a minimum RMS voltage corresponding to a time instant later than a time instant corresponding to the maximum RMS voltage. It should be understood that the second positive index indicates that a relative amplitude reduction value corresponding to the lead is greater than a fourth threshold. Conversely, a second negative index indicates that the relative amplitude reduction value corresponding to the lead is less than or equal to the fourth threshold. The quantity of the second positive index characterizes an overall degree of myocardial ischemia corresponding to the electrocardiogram signals in the stress state from the leads. That is, a large quantity of the second positive index indicates a great risk of myocardial ischemia.

In an embodiment, the initial average voltage is an average of initial voltages of the high-frequency waveform curves, and characterizes whether the overall cardiac pumping function corresponding to the electrocardiogram signals in the stress state from the leads is abnormal. The initial voltage refers to a voltage at an initial time instant in the high-frequency waveform curve. The maximum output power is determined based on the maximum of the root-mean-square voltages of the high-frequency waveform curves and characterizes an overall cardiac pumping capacity corresponding to the electrocardiogram signals in the stress state from the leads.

The second target lead is a lead whose the high-frequency waveform curve presents a target waveform. The target waveform includes a U wave, an L wave and a small V waves. A quantity of the second target lead characterizes a severity of coronary artery stenosis corresponding to the electrocardiogram signals in the stress state from the leads.

In this embodiment, the electrocardiogram signals in the stress state are acquired from the leads; then data processing is performed on the electrocardiogram signals in the stress state to obtain the respective high-frequency waveform curves; subsequently feature analysis is performed on the high-frequency waveform curves to obtain the dynamic high-frequency QRS waveform feature set, thus implementing the assessment and analysis by combining the static high-frequency QRS waveform feature set and the dynamic high-frequency QRS waveform feature set. As a result, the risk assessment score of heart failure is acquired more accurately by the electrocardiogram signals in the resting state and the electrocardiogram signals in the stress state from the leads, thereby improving the accuracy and convenience of the risk assessment of heart failure.

In an embodiment, as shown in FIG. 6, the step of assessing and analyzing the risk assessment feature set to obtain the risk assessment score of heart failure includes step 601.

In step 601, the risk assessment feature set is inputted into a preset risk assessment function or a pre-trained risk assessment network model to obtain the risk assessment score of heart failure.

The terminal may store the preset risk assessment function or the pre-trained risk assessment network model in advance, input the risk assessment feature set into the preset risk assessment function to output a risk assessment score of heart failure via the risk assessment function, and/or input the risk assessment feature set into the pre-trained risk assessment network model to output a risk assessment score of heart failure via the risk assessment network model.

In a specific example, a first risk assessment sub-score of heart failure is acquired based on the following equation:

$$f(t) = \begin{cases} 10 & (135ms < t < 149ms) \\ 5 & (120ms < t < 134ms) \\ 0 & (t < 120ms) \end{cases}$$

$f(t)$ represents the first risk assessment sub-score of heart failure; and $t$ represents the QRS duration.

A second risk assessment sub-score of heart failure is acquired based on the following equation:

$$\alpha(c) = c$$

$\alpha(c)$ represents the second risk assessment sub-score of heart failure; and $c$ represents a quantity of the first positive index.

A third risk assessment sub-score of heart failure is acquired based on the following equation:

$$\beta(v) = 5v$$

$\beta(v)$ represents the third risk assessment sub-score of heart failure; and $v$ represents a quantity of the first target lead.

A fourth risk assessment sub-score of heart failure is acquired based on the following equation:

$$\chi(b) = 3b$$

$\chi(b)$ represents the fourth risk assessment sub-score of heart failure; and $b$ represents a quantity of the target limb lead.

A fifth risk assessment sub-score of heart failure is acquired based on the following equation:

$$\delta(n) = n$$

$\delta(n)$ represents the fifth risk assessment sub-score of heart failure; and $n$ represents a quantity of the second positive index.

A sixth risk assessment sub-score of heart failure is acquired based on the following equation.

$$\varepsilon(m) = 10m \quad (m > 2)$$

$\varepsilon(m)$ represents the sixth risk assessment sub-score of heart failure; and $m$ represents the initial average voltage.

A seventh risk assessment sub-score of heart failure is acquired based on the following equation:

$$\phi(a) = \begin{cases} 10 & (a = 4) \\ 5 & (a = 6) \end{cases}$$

$\phi(a)$ represents the seventh risk assessment sub-score of heart failure; and $a$ represents the maximum output power.

An eighth risk assessment sub-score of heart failure is acquired based on the following equation:

$$\varphi(s) = 5s$$

$\varphi(s)$ represents the eighth risk assessment sub-score of heart failure; and $s$ represents a quantity of the second target lead.

The risk assessment score of heart failure is acquired based on the following equation of the risk assessment function:

$$y = f(t) + \alpha(c) + \beta(v) + \chi(b) + \delta(n) + \varepsilon(m) + \phi(a) + \varphi(s)$$

where $y$ represents the risk assessment score of heart failure; $f(t)$ represents the first risk assessment sub-score of heart failure; $\alpha(c)$ represents the second risk assessment sub-score of heart failure; $\beta(v)$ represents the third risk assessment sub-score of heart failure; $\chi(b)$ represents the fourth risk assessment sub-score of heart failure; $\delta(n)$ represents the fifth risk assessment sub-score of heart failure; $\varepsilon(m)$ represents the sixth risk assessment sub-score of heart failure; $\phi(a)$ represents the seventh risk assessment sub-score of heart failure; $\varphi(s)$ represents the eighth risk assessment sub-score of heart failure. Specific examples are described above, and may be flexibly applied according to user requirements in practical applications, which are not limited herein.

In a specific example, the eighth risk assessment sub-score of heart failure may further be acquired based on the following equation:

$$\varphi(s) = 5d + 2(s - d)$$

where $\varphi(s)$ represents the eighth risk assessment sub-score of heart failure; $s$ represents the quantity of the second target lead; $d$ represents a quantity of a third target lead; and $s - d$ represents a quantity of a fourth target lead. The third target lead is the second target lead for which a time instant corresponding to the minimum of the root-mean-square voltages of the high-frequency waveform curve is within a target time, and the target time starts from a starting time instant to a preset time instant of the electrocardiogram signal in the exercise state. It should be understood that the fourth target lead is the second target lead for which the time instant corresponding to the minimum of the root-mean-square voltages of the high-frequency waveform curve is outside the target time. In addition, the preset time instant may be but is not limited to 180 seconds, and is flexibly set according to user requirements in practical applications, which are not limited herein.

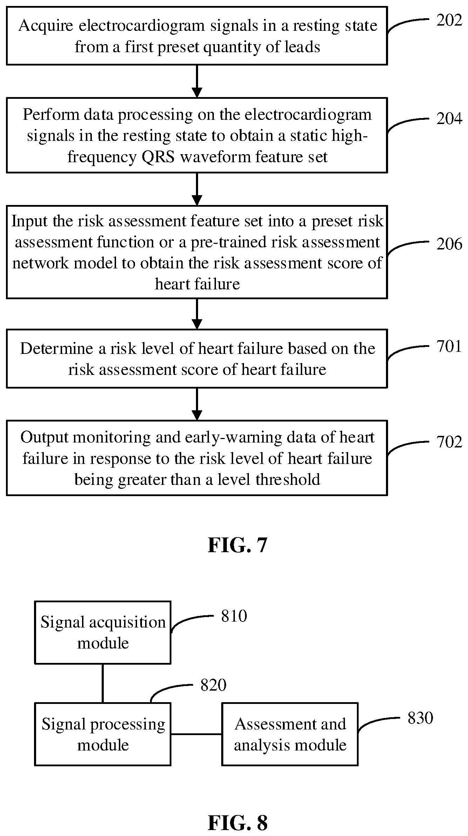

In an embodiment, as shown in FIG. 7, the above method further includes step 701 and step 702.

In step 701, a risk level of heart failure is determined based on the risk assessment score of heart failure.

In step 702, monitoring and early-warning data of heart failure is outputted in response to the risk level of heart failure being greater than a level threshold.

The terminal assesses and analyzes the risk assessment feature set to obtain the risk assessment score of heart failure, then determines the risk level of heart failure based on the risk assessment score of heart failure, and subsequently outputs the monitoring and early-warning data of heart failure in response to the risk levels of heart failure being greater than the level threshold.

In this embodiment, the risk level of heart failure is determined based on the risk assessment score of heart failure, then the monitoring and early-warning data of heart failure is outputted in response to the risk level of heart failure being greater than the level threshold to enable the object from which electrocardiogram signals are collected to promptly understand their own risk of heart failure, improving the convenience of the method for processing electrocardiogram signals.

In an embodiment, the step of determining the risk level of heart failure based on the risk assessment score of heart failure includes: determining the risk level of heart failure based on an interval where the risk assessment score of heart failure falls.

The above interval includes a first preset score interval, a second preset score interval and a third preset score interval. An upper limit of the first preset score interval is less than or equal to a lower limit of the second preset score interval, and an upper limit of the second preset score interval is less than or equal to a lower limit of the third preset score interval.

The risk level of heart failure includes a first level, a second level and a third level. The terminal determines the risk level of heart failure as the first level in a case that the risk assessment score of heart failure falls within the first preset score interval. The first level indicates the risk of heart failure of the object from which electrocardiogram signals are collected is relatively low, and an exercise rehabilitation is effective and the intensity of the exercise rehabilitation may be reduced. The terminal determines the risk level of heart failure as the second level in a case that the risk assessment score of heart failure falls within the second preset score interval. The second level indicates that the risk of heart failure of the object from which electrocardiogram signals are collected is moderate, and the exercise rehabilitation may be continued, and the intensity of the exercise rehabilitation is reduced when the risk assessment score of heart failure falls within the first preset score interval. The terminal determines the risk level of heart failure as the third level in a case that the risk assessment score of heart failure falls within the third preset score interval. The third level indicates that the risk of heart failure of the object from which electrocardiogram signals are collected is severe, exercise rehabilitation has suboptimal efficacy or the intensity of the exercise rehabilitation is temporarily reduced and gradually increased after adaptation.

In this embodiment, the risk level of heart failure is determined based on the interval where the risk assessment score of heart failure falls, so that the risk level of heart failure of the object from which electrocardiogram signals are collected may be accurately determined, facilitating the understanding of the effect of exercise rehabilitation and the adjustment of the intensity of the exercise rehabilitation.

It should be understood that although steps in the flowchart of FIG. 2 to FIG. 7 is displayed in sequence as indicated by the arrows, these steps are not necessarily executed in the sequence as indicated by the arrows. Unless otherwise explicitly specified herein, a sequence of performing the steps is not strictly limited, and the steps may be performed in other sequence. Moreover, at least some steps in FIG. 2 to FIG. 7 may include multiple sub-steps or multiple stages, which are unnecessarily performed at the same time, but may be performed at different times. The sequence of execution of these sub-steps or stages is unnecessarily performed sequentially, but may be performed in turn or alternately with other steps or at least a portion of sub-steps or stages of other steps.

In an embodiment, as shown in FIG. 8, an apparatus for processing electrocardiogram signals is provided. The apparatus includes a signal acquisition module 810, a signal processing module 820 and an assessment and analysis module 830.

The signal acquisition module 810 is configured to acquire electrocardiogram signals in a resting state from a first preset quantity of leads. The leads include a second preset quantity of limb leads, and the second preset quantity is less than the first preset quantity.

The signal processing module 820 is configured to perform data processing on the electrocardiogram signals in the resting state to obtain a static high-frequency QRS waveform feature set. The static high-frequency QRS waveform feature set includes a QRS duration, a quantity of a first positive index, a quantity of a first target lead and a quantity of a target limb lead. For each of the leads, the first positive index characterizes that a high-frequency morphological index corresponding to the lead is greater than a first threshold. The high-frequency morphological index is a ratio of a first total area to a second total area corresponding to the lead. The first total area is a total area of a reduced amplitude zone of the electrocardiogram signal in the resting state corresponding to the lead. The second total area is a total area under an envelope of the electrocardiogram signal in the resting state corresponding to the lead. The first target lead is a lead for which a total quantity of times the electrocardiogram signal in the resting state is in a downward trend exceeds a second threshold. The target limb lead is a limb lead for which a QRS complex voltage of the electrocardiogram signals in the resting state is less than a third threshold.

The assessment and analysis module 830 is configured to assess and analyze a risk assessment feature set to obtain a risk assessment score of heart failure. The risk assessment score of heart failure is used to determine a risk magnitude of heart failure. The risk assessment feature set includes the static high-frequency QRS waveform feature set.

In an embodiment, the risk assessment feature set further includes a dynamic high-frequency QRS waveform feature set. The signal acquisition module 810 is further configured to acquire the electrocardiogram signals in the stress state from the leads. The signal processing module 820 is further configured to perform data processing on the electrocardiogram signals in the stress state to obtain respective high-frequency waveform curves. The signal processing module 820 is further configured to perform feature analysis on the respective high-frequency waveform curves to obtain a dynamic high-frequency QRS waveform feature set. The dynamic high-frequency QRS waveform feature set includes a quantity of a second positive index, an initial average voltage, a maximum output power, and a quantity of a second target lead. The second positive index characterizes that a relative amplitude reduction value corresponding to each of the leads is greater than a fourth threshold. The relative amplitude reduction value is a ratio of an absolute amplitude value to a maximum RMS voltage. The absolute amplitude value is a difference between the maximum RMS voltage and a target RMS voltage. The maximum RMS voltage is a maximum of RMS voltages of the respective high-frequency waveform curves. The target RMS voltage is a minimum RMS voltage corresponding to a time instant later than a time instant corresponding to the maximum RMS voltage. The initial average voltage is an average of initial voltages of the high-frequency waveform curves. The maximum output power is determined based on the maximum of the root-mean-square voltages of the high-frequency waveform curves. The second target leads is a lead whose the high-frequency waveform curve presents a target waveform. The target waveform includes a U wave, an L wave and a small V wave.

In an embodiment, the signal processing module 820 includes a signal processing unit. The signal processing unit is configured to perform data extraction on the electrocardiogram signals in the resting state to obtain high-frequency components of static QRS waveforms corresponding to the leads. The signal processing unit is further configured to determine reduced amplitude zones corresponding to the leads based on the high-frequency components of the static QRS waveforms corresponding to the leads, and calculate a first total area and a second total area of each of the leads. The signal processing unit is further configured to acquire a high-frequency morphological index of each of the leads based on a ratio of the first total area to the second total area of the lead. The signal processing unit is further configured to count the quantity of the first positive index based on the high-frequency morphological indexes of all the leads and the first threshold.

In an embodiment, the signal processing unit is further configured to calculate an area of the reduced amplitude zone corresponding to each of the leads. The signal processing unit is further configured to count a quantity of a target area of the lead based on the area of the reduced amplitude zone corresponding to the lead. The target area refers to an area of the reduced amplitude zone that is greater than a fifth threshold. The signal processing unit is further configured to determine the quantity of a target area of the lead as the total quantity of times corresponding to the lead. The signal processing unit is further configured to determine the quantity of the first target lead based on the total quantities of times corresponding to all the leads and the second threshold.

In an embodiment, the assessment and analysis module 830 includes an assessment and analysis unit. The evaluation and analysis unit is configured to input the risk assessment feature set into a preset risk assessment function or a pre-trained risk assessment network model to obtain a risk assessment score of heart failure.

In an embodiment, the above apparatus further includes a risk grading module and a risk early-warning module.

The risk grading module is configured to determine a risk level of heart failure based on the risk assessment score of heart failure. The risk early-warning module is configured to output monitoring and early-warning data of heart failure in response to the risk level of heart failure being greater than a level threshold.

For details about the apparatus for processing electrocardiogram signals, reference can be made to above descriptions of the method for processing electrocardiogram signals, and thus the apparatus is not repeated herein. The modules in the above apparatus for processing electrocardiogram signals may be implemented entirely or partially by software, hardware, or a combination thereof. The modules may be embodied in or independent of a processor of a computer device in a hardware form, or may be stored in a memory of a computer device in a software form, facilitating the processor invoking and performing operations corresponding to the modules.

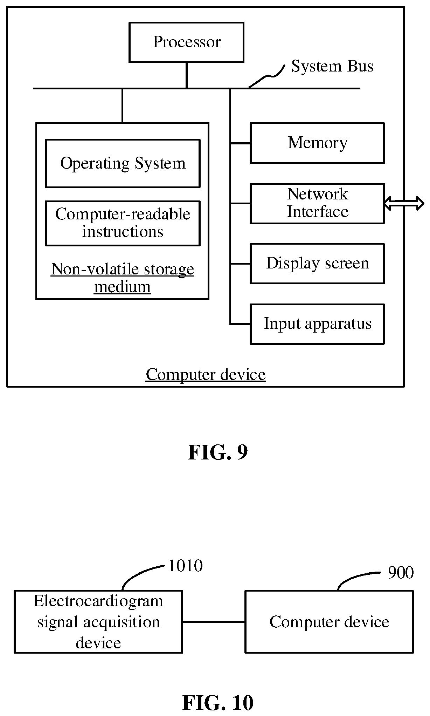

In an embodiment, a computer device 900 is provided, and the computer device 900 may be a terminal, and its internal structure diagram may be referred to FIG. 9. The computer device 900 includes a processor, a memory, a network interface, a display screen and an input apparatus that are connected to each other via a system bus. The processor of the computer device 900 is configured to provide computing and control functions. The memory of the computer device 900 includes a non-volatile storage medium and an internal memory. The non-volatile storage medium stores an operating system and computer-readable instructions. The internal memory provides an environment for running of the operating system and the computer-readable instructions in the non-volatile storage medium. The network interface of the computer device 900 is configured to communicate with an external terminal via network connection. The computer-readable instructions are executed by the processor to implement a method for processing electrocardiogram signals. The display screen of the computer device 900 may be a liquid crystal display screen or an electronic ink display screen. The input apparatus of the computer device 900 may be a touch layer covered on the display screen, may also be a key, a trackball or a touchpad that is provided on a housing of the computer device 900, and may further be an external keyboard, a touchpad, a mouse, or the like.

It can be understood by those skilled in the art that, the structure shown in FIG. 9 is only a block diagram of a part of a structure correlated to the solutions according to the present disclosure and does not constitute a limitation to the computer device 900 to which the solutions according to the present disclosure are applied. The computer device 900 may include more components or fewer components than the components shown in the figure, or some components may be combined, or a different arrangement of components may be adopted.

In a third aspect, a computer device 900 is provided. The computer device includes a memory and a processor. The memory stores computer-readable instructions, and the computer-readable instructions, when being executed by the processor, perform the method according to any one of the embodiments of the method described above.

In a fourth aspect, a system for processing electrocardiogram signals is provided. As shown in FIG. 10, the system includes an electrocardiogram signal acquisition device 1010 and the computer device 900 according to any one of the embodiments of the device described above.

The electrocardiogram signal acquisition device 1010 is electrically connected to the computer device 900 and is configured to acquire electrocardiogram signals in the resting state.

In this embodiment, the risk assessment score of heart failure can be directly acquired through a risk assessment system of heart failure, that is, the risk magnitude of heart failure can be accurately assessed. Furthermore, directly assessing the risk magnitude of heart failure through the risk assessment score of heart failure avoids the joint risk assessment of heart failure using multiple detection data in conventional methods, reducing the time consumption of the risk assessment of heart failure and improving the efficiency of the risk assessment of heart failure.

In an embodiment, the electrocardiogram signal acquisition device 1010 is further configured to acquire electrocardiogram signals in a stress state.

In a fifth aspect, a computer-readable storage medium is provided. The computer-readable storage medium stores computer-readable instructions. The computer-readable instructions, when being executed by a processor, perform the method according to any one of the above embodiments of the method.

Those skilled in the art may understand that all or some of the procedures of the methods of the above embodiments may be implemented by computer-readable instructions instructing relevant hardware. The computer-readable instructions may be stored in a non-volatile computer-readable storage medium. When the computer-readable instructions are executed, the procedures of the embodiments of the above methods may be included. Any reference to a memory, a storage, a database, or another medium used in the embodiments provided in this present disclosure may include a non-volatile and/or volatile memory. The non-volatile memory may include a read-only memory (ROM), a programmable ROM (PROM), an electrically programmable ROM (EPROM), an electrically erasable programmable ROM (EEPROM), or a flash memory. The volatile memory may include a random access memory (RAM) or an external high-speed cache. As an illustration instead of a limitation, the RAM is available in multiple forms, such as a static RAM (SRAM), a dynamic RAM (DRAM), a synchronous DRAM (SDRAM), a double data rate SDRAM (DDRSDRAM), an enhanced SDRAM (ESDRAM), a synchronous link (Synchlink) DRAM (SLDRAM), a Rambus direct RAM (RDRAM), a direct Rambus dynamic RAM (DRDRAM), and a Rambus dynamic RAM (RDRAM).

Technical features in the above embodiments may be arbitrarily combined. For brief description, not all possible combinations of the technical features are described herein. However, all combinations of the technical features are regarded as being within the scope of the present disclosure, as long as there is no conflict.

The above embodiments show only several implementations of the present disclosure. The embodiments are described specifically with details, and shall not be construed as a limitation to a protection scope of the present disclosure. It should be noted that for those skilled in the art, modifications and improvements can be made without deviating from the concept of the present disclosure, and the modifications and improvements fall within the protection scope of the present disclosure. The protection scope of the present disclosure is determined by the appended claims.

**Claims**

1.  A method for processing electrocardiogram signals, comprising:

    acquiring electrocardiogram signals in a resting state from a first preset quantity of leads; wherein the leads comprise a second preset quantity of limb leads; and the second preset quantity is less than the first preset quantity;

    performing data processing on the electrocardiogram signals in the resting state to obtain a static high-frequency QRS waveform feature set; wherein the static high-frequency QRS waveform feature set comprises a QRS duration, a quantity of a first positive index, a quantity of a first target lead and a quantity of a target limb lead; for each of the leads, the first positive index is for characterizing that a high-frequency morphological index corresponding to the lead is greater than a first threshold; the high-frequency morphological index is a ratio of a first total area to a second total area corresponding to the lead; the first total area is a total area of a reduced amplitude zone of the electrocardiogram signal in the resting state corresponding to the lead; the second total area is a total area under an envelope of the electrocardiogram signal in the resting state corresponding to the lead; the first target lead is a lead for which a total quantity of times the electrocardiogram signal in the resting state is in a downward trend exceeds a second threshold, and the target limb lead is a limb lead for which a QRS complex voltage of the electrocardiogram signals in the resting state is less than a third threshold;

    assessing and analyzing a risk assessment feature set to obtain a risk assessment score of heart failure, wherein the risk assessment score of heart failure is for determining a risk magnitude of heart failure, and the risk assessment feature set comprises the static high-frequency QRS waveform feature set;

    wherein the performing data processing on the electrocardiogram signals in the resting state to obtain the static high-frequency QRS waveform feature set comprises:

    for each of the leads,

        performing data extraction on the electrocardiogram signal in the resting state to obtain a high-frequency component of a static QRS waveform corresponding to the lead;

        determining a reduced amplitude zone corresponding to the lead based on the high-frequency component of the static QRS waveform, and calculating the first total area and the second total area corresponding to the lead;

        acquiring a high-frequency morphological index of the lead based on a ratio of the first total area to the second total area;

        counting the quantity of the first positive index based on the high-frequency morphological indexes of all the leads and the first threshold;

        calculating an area of the reduced amplitude zone corresponding to the lead; counting a quantity of a target area of the lead based on the area of the reduced amplitude zone corresponding to the lead; wherein the target area refers to an area of the reduced amplitude zone that is greater than a fifth threshold;

        determining the quantity of the target area of the lead as the total quantity of times corresponding to the lead; and

        determining the quantity of the first target lead based on the total quantities of times corresponding to all the leads and the second threshold.

2.  The method according to claim 1, wherein the risk assessment feature set further comprises a dynamic high-frequency QRS waveform feature set, and the method further comprises:

    acquiring electrocardiogram signals in a stress state from the leads;

    performing data processing on the electrocardiogram signals in the stress state to obtain respective high-frequency waveform curves;

    performing feature analysis on the respective high-frequency waveform curves to obtain the dynamic high-frequency QRS waveform feature set, wherein the dynamic high-frequency QRS waveform feature set comprises at least two of a quantity of a second positive index, an initial average voltage, a maximum output power, and a quantity of a second target lead; the second positive index is for characterizing that a relative amplitude reduction value corresponding to each of the leads is greater than a fourth threshold; the relative amplitude reduction value is a ratio of an absolute amplitude value to a maximum RMS voltage; the absolute amplitude value is a difference between the maximum RMS voltage and a target RMS voltage; the maximum RMS voltage is a maximum of RMS voltages of each of the respective high-frequency waveform curves; the target RMS voltage is a minimum RMS voltage corresponding to a time instant later than a time instant corresponding to the maximum RMS voltage; the initial average voltage is an average of initial voltages of the respective high-frequency waveform curves; the maximum output power is determined based on the maximum of the root-mean-square voltages of the high-frequency waveform curves; the second target lead is a lead whose the high-frequency waveform curve presents a target waveform; and the target waveform comprises a U wave, an L wave and a small V wave.

3. The method according to any one of claims 1 to 2, wherein the assessing and analyzing the risk assessment feature set to obtain the risk assessment score of heart failure comprises:
inputting the risk assessment feature set into a preset risk assessment function or a pre-trained risk assessment network model to obtain the risk assessment score of heart failure.

4. The method according to any one of claims 1 to 2, further comprising:

determining a risk level of heart failure based on the risk assessment score of heart failure; and
outputting monitoring and early-warning data of heart failure in response to the risk level of heart failure being greater than a level threshold.

5. An apparatus for processing electrocardiogram signals, comprising:

a signal acquisition module, configured to acquire electrocardiogram signals in a resting state from a first preset quantity of leads, wherein the leads comprise a second preset quantity of limb leads, and the second preset quantity is less than the first preset quantity;
a signal processing module, configured to perform data processing on the electrocardiogram signals in the resting state to obtain a static high-frequency QRS waveform feature set, wherein the static high-frequency QRS waveform feature set comprises a QRS duration, a quantity of a first positive index, a quantity of a first target lead and a quantity of a target limb lead; for each of the leads, the first positive index is for characterizing that a high-frequency morphological index corresponding to the lead is greater than a first threshold; the high-frequency morphological index is a ratio of a first total area to a second total area corresponding to the lead; the first total area is a total area of a reduced amplitude zone of the electrocardiogram signal in the resting state corresponding to the lead; the second total area is a total area under an envelope of the electrocardiogram signal in the resting state corresponding to the lead; the first target lead is a lead for which a total quantity of times the electrocardiogram signal in the resting state is in a downward trend exceeds a second threshold, and the target limb lead is a limb lead for which a QRS complex voltage of the electrocardiogram signals in the resting state is less than a third threshold; and
an assessment and analysis module, configured to assess and analyze a risk assessment feature set to obtain a risk assessment score of heart failure, wherein the risk assessment score of heart failure is for determining a risk magnitude of heart failure, and the risk assessment feature set comprises the static high-frequency QRS waveform feature set;
wherein the signal processing module comprises a signal processing unit; the signal processing unit is configured to perform, for each of the leads, data extraction on the electrocardiogram signal in the resting state to obtain a high-frequency component of a static QRS waveform corresponding to the lead; the signal processing unit is further configured to determine a reduced amplitude zone corresponding to the lead based on the high-frequency component of the static QRS waveform and calculate a first total area and a second total area corresponding to the lead; the signal processing unit is further configured to acquire a high-frequency morphological index of the lead based on a ratio of the first total area to the second total area; the signal processing unit is further configured to count the quantity of the first positive index based on the high-frequency morphological indexes of all the leads and the first threshold; the signal processing unit is further configured to calculate an area of the reduced amplitude zone corresponding to the lead; the signal processing unit is further configured to count a quantity of a target area of the lead based on the area of the reduced amplitude zone corresponding to the lead; where the target area refers to an area of the reduced amplitude zone that is greater than a fifth threshold; the signal processing unit is further configured to determine the quantity of the target area of the lead as the total quantity of times corresponding to the lead; and the signal processing unit is further configured to determine the quantity of the first target lead based on the total quantities of times corresponding to all the leads and the second threshold.

6. The apparatus according to claim 5, wherein the risk assessment feature set further comprises a dynamic high-frequency QRS waveform feature set, the signal acquisition module is further configured to acquire electrocardiogram signals in a stress state from the leads; the signal processing module is further configured to perform data processing on the electrocardiogram signals in the stress state to obtain respective high-frequency waveform curves and perform feature analysis on the respective high-frequency waveform curves to obtain the dynamic high-frequency QRS waveform feature set, wherein the dynamic high-frequency QRS waveform feature set comprises at least two of a quantity of a second positive index, an initial average voltage, a maximum output power, and a quantity of a second target lead; the second positive index is for characterizing that a relative amplitude reduction value corresponding to each of the leads is greater than a fourth threshold; the relative amplitude reduction value is a ratio of an absolute amplitude value to a maximum RMS voltage; the absolute amplitude value is a difference between the maximum RMS voltage and a target RMS voltage; the maximum RMS voltage is a maximum of RMS voltages of each of the respective high-frequency waveform curves; the target RMS voltage is a minimum RMS voltage corresponding to a time instant later than a time instant corresponding to the maximum RMS voltage; the initial average voltage is an average of initial voltages of the respective high-frequency waveform curves; the maximum output power is determined based on the maximum of the root-mean-square voltages of the high-frequency waveform curves; the second target lead is a lead whose the high-frequency waveform curve presents a target waveform; and the target waveform comprises a U wave, an L wave and a small V wave.

7. The apparatus according to any one of claims 5 to 6, wherein the assessment and analysis module comprises an assessment and analysis unit; and the evaluation and analysis unit is configured to input the risk assessment feature set into a preset risk assessment function or a pre-trained risk assessment network model to obtain the risk assessment score of heart failure.

8. The apparatus according to any one of claims 5 to 6, further comprising a risk grading module and a risk early-warning module, wherein

the risk grading module is configured to determine a risk level of heart failure based on the risk assessment score of heart failure; and
the risk early-warning module is configured to output monitoring and early-warning data of heart failure in response to the risk level of heart failure being greater than a level threshold.

9. A computer device, comprising a memory, a processor and computer-readable instructions stored in the memory and executable by the processor, wherein the computer-readable instructions, when being executed by the processor, perform the method according to any one of claims 1 to 4.

10. A system for processing electrocardiogram signals, comprising an electrocardiogram signal acquisition device and the computer device according to claim 9, wherein the electrocardiogram signal acquisition device is electrically connected to the computer device and is configured to acquire electrocardiogram signals in the resting state.

11. A computer-readable storage medium storing computer-executable instructions, wherein the computer-readable instructions, when being executed by a processor, perform the method according to any one of claims 1 to 4.

**FIG. 1**

| | |
|---|---|
| Acquire electrocardiogram signals in a resting state from a first preset quantity of leads | 202 |

| | |
|---|---|
| Perform data processing on the electrocardiogram signals in the resting state to obtain a static high-frequency QRS waveform feature set | 204 |

| | |
|---|---|
| Assess and analyze a risk assessment feature set to obtain a risk assessment score of heart failure | 206 |

**FIG. 2**

| Perform data extraction on the electrocardiogram signal in resting state to obtain a high-frequency component of a static QRS waveform corresponding to each lead | — 301 |

| Determine a reduced amplitude zone corresponding to the lead based on the high-frequency component of the static QRS waveform, and calculate a first total area and a second total area corresponding to the lead | — 302 |

| Acquire a high-frequency morphological index of the lead based on a ratio of the first total area to the second total area | — 303 |

| Count the quantity of the first positive index based on the high-frequency morphological indexes of all the leads and the first threshold | — 304 |

**FIG. 3**

Perform data extraction on the electrocardiogram signals in the resting state to obtain high-frequency components of static QRS waveforms corresponding to the leads — 301

Determine a reduced amplitude zone corresponding to the lead based on the high-frequency component of the static QRS waveform, and calculate a first total area and a second total area corresponding to the lead — 302

Calculate an area of the reduced amplitude zone corresponding to the lead — 401

Count a quantity of a target area of the lead based on the area of the reduced amplitude zone corresponding to the lead — 402

Acquire a high-frequency morphological index of the lead based on a ratio of the first total area to the second total area — 303

Determine the quantity of the target area as the total quantity of times corresponding to lead — 403

Count the quantity of the first positive index based on the high-frequency morphological indexes of all the leads and the first threshold — 304

Determine the quantity of the first target lead based on the total quantities of times corresponding to all leads and second threshold — 404

FIG. 4

Acquire electrocardiogram signals in a stress state from leads — 501

Perform data processing on the electrocardiogram signals in the stress state to obtain respective high-frequency waveform curves — 502

Acquire electrocardiogram signals in a resting state from a first preset quantity of leads — 202

Perform feature analysis on the high-frequency waveform curves to obtain a dynamic high-frequency QRS waveform feature set — 503

Perform data processing on the electrocardiogram signals in the resting state to obtain a static high-frequency QRS waveform feature set — 204

Assess and analyze a risk assessment feature set to obtain a risk assessment score of heart failure — 206

**FIG. 5**

Acquire electrocardiogram signals in a resting state from a first preset quantity of leads — 202

Perform data processing on the electrocardiogram signals in the resting state to obtain a static high-frequency QRS waveform feature set — 204

Input the risk assessment feature set into a preset risk assessment function or a pre-trained risk assessment network model to obtain the risk assessment score of heart failure — 601

**FIG. 6**

```
┌─────────────────────────────────────────┐
│ Acquire electrocardiogram signals in a    │ ─── 202
│ resting state from a first preset quantity │
│ of leads                                   │
└─────────────────────────────────────────┘
                    │
                    ▼
┌─────────────────────────────────────────┐
│ Perform data processing on the            │ ─── 204
│ electrocardiogram signals in the resting  │
│ state to obtain a static high-frequency    │
│ QRS waveform feature set                   │
└─────────────────────────────────────────┘
                    │
                    ▼
┌─────────────────────────────────────────┐
│ Input the risk assessment feature set into │ ─── 206
│ a preset risk assessment function or a     │
│ pre-trained risk assessment network model  │
│ to obtain the risk assessment score of     │
│ heart failure                              │
└─────────────────────────────────────────┘
                    │
                    ▼
┌─────────────────────────────────────────┐
│ Determine a risk level of heart failure    │ ─── 701
│ based on the risk assessment score of      │
│ heart failure                              │
└─────────────────────────────────────────┘
                    │
                    ▼
┌─────────────────────────────────────────┐
│ Output monitoring and early-warning data   │ ─── 702
│ of heart failure in response to the risk   │
│ level of heart failure being greater than  │
│ a level threshold                          │
└─────────────────────────────────────────┘
```

**FIG. 7**

```
┌──────────────────┐
│ Signal acquisition│ ─── 810
│ module            │
└──────────────────┘
         │
         │        ─── 820
┌──────────────────┐        ┌──────────────────┐
│ Signal processing │        │ Assessment and    │ ─── 830
│ module            │────────│ analysis module   │
└──────────────────┘        └──────────────────┘
```

**FIG. 8**

Processor

System Bus

Operating System

Memory

Computer-readable
instructions

Network
Interface

Non-volatile storage
medium

Display screen

Input apparatus

Computer device

**FIG. 9**

1010

900

Electrocardiogram
signal acquisition
device

Computer device

**FIG. 10**

**FIG. 11**

## INTERNATIONAL SEARCH REPORT

| International application No. |
| --- |
| **PCT/CN2023/093494** |

| **A.** | **CLASSIFICATION OF SUBJECT MATTER** |
| --- | --- |

G16H50/30(2018.01)i; A61B5/366(2021.01)i; A61B5/346(2021.01)i; A61B5/00(2006.01)i

According to International Patent Classification (IPC) or to both national classification and IPC

| **B.** | **FIELDS SEARCHED** |
| --- | --- |

Minimum documentation searched (classification system followed by classification symbols)

IPC:G06H,A61B

Documentation searched other than minimum documentation to the extent that such documents are included in the fields searched

Electronic data base consulted during the international search (name of data base and, where practicable, search terms used)

CNTXT, ENTXT, VCN, CJFD, IEEE: 分析, 风险, 评估, 评价, 心电, 心律, 心衰, 信号, QRS, ECG, electrocardiography, heartbeat, heart rate, heart failure, evaluate, analysis, risk

| **C.** | **DOCUMENTS CONSIDERED TO BE RELEVANT** | | |
| --- | --- | --- | --- |
| Category* | Citation of document, with indication, where appropriate, of the relevant passages | | Relevant to claim No. |
| PX | CN 115602329 A (BISHENGPU BIOTECHNOLOGY CO., LTD.) 13 January 2023 (2023-01-13) <br> claims 1-10, and description, paragraphs [0088]-[0097] | | 1-11 |
| A | CN 113080991 A (TAIYUAN UNIVERSITY OF TECHNOLOGY) 09 July 2021 (2021-07-09) <br> description, paragraphs [0048]-[0061] | | 1-11 |
| A | CN 113712569 A (BISHENGPU BIOTECHNOLOGY CO., LTD.) 30 November 2021 (2021-11-30) <br> entire document | | 1-11 |
| A | CN 110063725 A (SHANGHAI ZHANGMEN TECHNOLOGY CO., LTD.) 30 July 2019 (2019-07-30) <br> entire document | | 1-11 |
| A | CN 111281372 A (MOSHENG (BEIJING) MEDICAL EQUIPMENT CO., LTD.) 16 June 2020 (2020-06-16) <br> entire document | | 1-11 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **11 August 2023** | **18 August 2023** |

| Name and mailing address of the ISA/CN | Authorized officer |
| --- | --- |
| **China National Intellectual Property Administration (ISA/CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/093494**

**C.    DOCUMENTS CONSIDERED TO BE RELEVANT**

| Category* | Citation of document, with indication, where appropriate, of the relevant passages | Relevant to claim No. |
|---|---|---|
| A | US 2020260979 A1 (LEPU MEDICAL TECHNOLOGY (BEJING) CO., LTD.) 20 August 2020 (2020-08-20) entire document | 1-11 |

## INTERNATIONAL SEARCH REPORT
### Information on patent family members

International application No.

**PCT/CN2023/093494**

| Patent document cited in search report | | | Publication date (day/month/year) | Patent family member(s) | | | Publication date (day/month/year) |
|---|---|---|---|---|---|---|---|
| CN | 115602329 | A | 13 January 2023 | None | | | |
| CN | 113080991 | A | 09 July 2021 | None | | | |
| CN | 113712569 | A | 30 November 2021 | None | | | |
| CN | 110063725 | A | 30 July 2019 | None | | | |
| CN | 111281372 | A | 16 June 2020 | None | | | |
| US | 2020260979 | A1 | 20 August 2020 | EP | 3692900 | A1 | 12 August 2020 |
| | | | | EP | 3692900 | A4 | 14 July 2021 |
| | | | | WO | 2019100566 | A1 | 31 May 2019 |
| | | | | US | 11344243 | B2 | 31 May 2022 |
| | | | | CN | 107714023 | A | 23 February 2018 |

Form PCT/ISA/210 (patent family annex) (July 2022)

REFERENCES CITED IN THE DESCRIPTION

**Patent documents cited in the description**

- CN 2022114516528